# EUROPEAN PATENT APPLICATION

(11) **EP 4 438 026 A1**
(43) Date of publication of application: **02.10.2024**
(21) Application number: 22968572.2
(22) Date of filing: 16.12.2022
(51) Int. Cl.: A61K 6/802, A61C 5/70, A61C 13/00, A61C 13/083, A61K 6/17, A61K 6/804, C04B 35/117

(54) **DENTAL-USE ALUMINA PRE-SINTERED BODY THAT BECOMES HIGHLY TRANSLUCENT ALUMINA SINTERED BODY**

(71) Applicant: Kuraray Noritake Dental Inc., Kurashiki-shi, Okayama 710-0801 (JP)
(72) Inventor: NAKANO Kirihiro, Miyoshi-shi, Aichi 470-0293 (JP); SAKAMOTO Hiroyuki, Miyoshi-shi, Aichi 470-0293 (JP); KASHIKI Nobusuke, Miyoshi-shi, Aichi 470-0293 (JP); KATO Shinichiro, Miyoshi-shi, Aichi 470-0293 (JP); ISHINO Hiroshige, Miyoshi-shi, Aichi 470-0293 (JP)
(74) Representative: D Young & Co LLP
(86) International application number: PCT/JP2022/046495
(87) International publication number: WO 2024/127664

(57) **Abstract**

The present invention provides an alumina pre-sintered body that demonstrates reduced yellowing after sintering, exhibits high translucency and high linear light transmittance in the sintered body even when sintered under atmospheric pressure, and yields an alumina sintered body with high aesthetics suitable for dental applications. The present invention relates to an alumina pre-sintered body for dental use comprising alumina particles with an average primary particle diameter of 30 to 300 nm, a sintering aid, and a bluish colorant, wherein the content of the sintering aid is 10 to 5,000 ppm.

## Description

### TECHNICAL FIELD

The present invention relates to a pre-sintered body comprising aluminum oxide (alumina) as its main component, and that, upon firing, transforms into a sintered body that exhibits high transparency. The invention also relates to a sintered body with high aesthetic qualities, and to a method for producing such a pre-sintered body.

### BACKGROUND ART

Over the last years, sintered bodies of metal oxides have become prevalent as dental materials. Concerning the appearance of sintered bodies, those with adjusted shades and translucency are used to match the appearance of healthy teeth remaining around the patient's treated area. Generally, sintered bodies are adjusted to provide higher transparency toward the incisal edge, and more intense color toward the cervical region.

Glass, zirconia, and aluminum oxide represent examples of metal oxides used in dental materials (for example, Patent Literatures 1 and 2).

Glass offers high transparency but exhibits weak tensile strength, while zirconia is high in strength but lacks sufficient transparency. Concerning aluminum oxide, it can yield a material with high strength and high translucency with the use of specialized firing equipment such as in HIP (Hot Isostatic Pressing) and hydrogen firing. However, this approach is industrially disadvantageous in terms of production costs.

### CITATION LIST

### Patent Literature

Patent Literature 1: WO2019/131782
Patent Literature 2: JP 2001-213664 A

### SUMMARY OF INVENTION

### Technical Problem

For example, Patent Literature 1 discloses a zirconia sintered body with varying shades of color, and it describes zirconia sintered bodies with unchanging patterns of increase or decrease in L*a*b* values along the shading direction.

However, although the zirconia sintered bodies of Patent Literature 1 demonstrate excellent shade, the low linear light transmittance of zirconia materials leaves room for improvement in terms of transparency at the incisal edge. When transparency is taken into account, it is difficult to say that zirconia alone fully meets the aesthetic requirements adequate for dental purposes, particularly in achieving a more natural tooth appearance.

Patent Literature 2 discloses an alumina sintered body described as having aesthetics suited for dental applications. While this related art discloses L*a*b* values, it does not disclose the ΔL* value, an index of translucency. Moreover, because this related art uses an alumina powder with a large median diameter D50 of 0.45 µm or greater, it fails to achieve a high linear light transmittance.

There is also an issue where adding sintering aids to the alumina powder and firing it under atmospheric pressure causes yellowing and reduces translucency. Patent Literature 2 does not address this yellowing issue, and it cannot be said as achieving aesthetics suited for dental applications.

As discussed above, there have not been thorough studies regarding pre-sintered bodies that could yield alumina sintered bodies with high linear light transmittance and reduced yellow tint when sintered under atmospheric pressure without HIP. That is, sufficient investigations have not been conducted concerning pre-sintered bodies that could transform into highly aesthetic sintered bodies suited for dental applications, particularly for the tip (incisal edge) of teeth.

It is accordingly an object of the present invention to provide an alumina pre-sintered body that demonstrates reduced yellowing in the sintered body after sintering, exhibits high translucency and high linear light transmittance in the sintered body even when sintered under atmospheric pressure, and yields an alumina sintered body with high aesthetics suitable for dental applications.

### Solution to Problem

The present inventors conducted intensive studies to find a solution to the foregoing issues, and found that an alumina pre-sintered body having an average primary particle diameter of 30 to 300 nm, and comprising 10 to 5,000 ppm of a sintering aid with a bluish colorant provides high aesthetic qualities by exhibiting excellent translucency and linear light transmittance after sintering under atmospheric pressure. This led to the completion of the present invention after further examinations.

Specifically, the present invention includes the following.
[1] An alumina pre-sintered body for dental use, comprising alumina particles with an average primary particle diameter of 30 to 300 nm, a sintering aid, and a bluish colorant,
   wherein the content of the sintering aid is 10 to 5,000 ppm.
[2] The alumina pre-sintered body for dental use according to [1], wherein the bluish colorant comprises a cobalt component.
[3] The alumina pre-sintered body for dental use according to [2], wherein the content of the cobalt component is 60 ppm or less.
[4] The alumina pre-sintered body for dental use according to [2] or [3], wherein the cobalt component is a component derived from a salt and/or a complex.
[5] The alumina pre-sintered body for dental use according to any one of [1] to [4], which shows a linear light transmittance of 0.8% or more as a sintered body of 1.0 mm thickness fired under atmospheric pressure without a hot isostatic pressing process.
[6] The alumina pre-sintered body for dental use according to any one of [1] to [5], which shows a b* value of -8.0 to 14.2 as a sintered body of 1.2 mm thickness fired under atmospheric pressure without a hot isostatic pressing process.
[7] The alumina pre-sintered body for dental use according to any one of [1] to [6], wherein the sintering aid comprises at least one element selected from the group consisting of a Group 2 element, Ce, Zr, and Y
[8] The alumina pre-sintered body for dental use according to any one of [1] to [7], wherein the alumina particles comprise α-alumina with a purity of 99.5% or more.
[9] A dental mill blank comprising a portion with the alumina pre-sintered body of any one of [1] to [8].
[10] The dental mill blank according to [9], wherein said portion is a portion with the highest translucency.
[11] A method for producing an alumina pre-sintered body for dental use, comprising the step of allowing a bluish colorant to uniformly adhere to alumina particles,
   wherein the alumina pre-sintered body for dental use comprises alumina particles with an average primary particle diameter of 30 to 300 nm, a sintering aid, and a bluish colorant, and
   the content of the sintering aid is 10 to 5,000 ppm.
[12] The method for producing an alumina pre-sintered body for dental use according to [11], wherein the step of allowing a bluish colorant to uniformly adhere to alumina particles comprises the step of dispersing α-alumina particles in a dispersion medium with the bluish colorant dissolved in the dispersion medium.
[13] The method for producing an alumina pre-sintered body for dental use according to [11] or [12], which comprises firing at 600°C or more and 1,200°C or less under atmospheric pressure.
[14] An alumina sintered body for dental use having an average crystal grain size of 0.3 to 8.0 µm and comprising a sintering aid and a bluish colorant,
   wherein the content of the sintering aid is 10 to 5,000 ppm.
[15] The alumina sintered body for dental use according to [14], wherein the bluish colorant comprises a cobalt component.
[16] The alumina sintered body for dental use according to [15], wherein the content of the cobalt component is 60 ppm or less.
[17] The alumina sintered body for dental use according to [15] or [16], wherein the cobalt component is a component derived from a salt and/or a complex.
[18] The alumina sintered body for dental use according to any one of [14] to [17], which has a linear light transmittance of 0.8% or more at a thickness of 1.0 mm.
[19] The alumina sintered body for dental use according to any one of [14] to [18], which has a b* value of -8.0 to 14.2 as a sintered body of 1.2 mm thickness.
[20] The alumina sintered body for dental use according to any one of [14] to [19], wherein the sintering aid comprises at least one element selected from the group consisting of a Group 2 element, Ce, Zr, and Y

### Advantageous Effects of Invention

According to the present invention, an alumina pre-sintered body for dental use can be provided that demonstrates reduced yellowing in the sintered body after sintering, exhibits high translucency and high linear light transmittance in the sintered body even when sintered under atmospheric pressure, and yields an alumina sintered body with high aesthetics suitable for dental applications.

The present invention can also provide an alumina pre-sintered body for dental use that transforms into highly aesthetic alumina sintered bodies suited for the incisal edge of incisors or canines in particular.

### BRIEF DESCRIPTION OF DRAWINGS

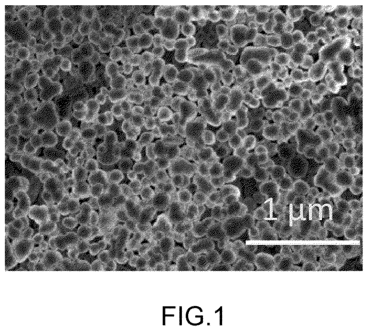

[FIG. 1] An electron micrograph of a pre-sintered body according to Example 1.

### DESCRIPTION OF EMBODIMENTS

An alumina pre-sintered body of the present invention comprises alumina particles with an average primary particle diameter of 30 to 300 nm, a sintering aid, and a bluish colorant, wherein the content of the sintering aid is 10 to 5,000 ppm.

The following describes a pre-sintered body of the present invention.

The pre-sintered body can be a precursor (intermediate product) of a sintered body.

In this specification, "pre-sintered body" refers to a state where particles of alumina have formed necks, and have solidified without being fully sintered.

The pre-sintered body may have a predetermined shape (for example, a disc shape or cuboidal shape).

The pre-sintered body may be a workpiece that has been processed into, for example, a crown shape. The pre-sintered body will be referred to as "workpiece", or "cut or ground workpiece" when it has been processed. The workpiece can be obtained by, for example, processing an alumina disc into a dental product (for example, a crown-shaped prosthesis) with the CAD/CAM (Computer-Aided Design/Computer-Aided Manufacturing) system.

A pre-sintered body of the present invention comprises necks formed by particles of alumina (hereinafter, also referred to simply as "alumina particles"), and the extent of necking varies with the average particle diameter of the alumina particles, resulting in a change in the hardness of the pre-sintered body. The smaller the average primary particle diameter of the alumina particles, the more likely necking takes place during pre-sintering.

In view of enhancing strength after sintering, and providing high aesthetic qualities in particular, an alumina pre-sintered body of the present invention comprises a sintering aid (an auxiliary agent that accelerates and stabilizes sintering of alumina).

The sintering aid contained in an alumina pre-sintered body of the present invention preferably comprises at least one element selected from the group consisting of a Group 2 element (Be, Mg, Ca, Sr, Ba, Ra), Ce, Zr, and Y, more preferably at least one element selected from the group consisting of Mg, Ca, Sr, Ba, Ce, Zr, and Y, even more preferably at least one element selected from the group consisting of Mg, Ce, Zr, and Y

The sintering aid is most preferably a magnesium compound.

Examples of the magnesium compound include oxides, nitrates, acetates, hydroxides, and chlorides.

Examples of the sintering aid include MgCl₂, Mg(OH)₂, CeO₂, ZrO₂, and Y₂O₃.

The magnesium compound as a sintering aid is not limited, as long as it is a magnesium compound that turns into an oxide at 1,200°C or less during a sintering process in the atmosphere. Preferred examples include magnesium nitrate, magnesium chloride, magnesium hydroxide, and magnesium acetate. The sintering aid may be used alone, or two or more thereof may be used in combination.

Generally, the content of the sintering aid in a raw material alumina powder according to the present invention is preferably 10 ppm to 5,000 ppm, more preferably 20 ppm to 3,000 ppm, even more preferably 30 ppm to 1,500 ppm in terms of the aforementioned elements (for example, in terms of a Mg element). In this specification, ppm means ppm by mass.

When the sintering aid content falls within these ranges, the sintering aid integrates with other constituents, allowing the sintered body to maintain high translucency and linear light transmittance, as well as high strength, even when sintered under atmospheric pressure.

When the content of the sintering aid (preferably, a magnesium compound) is low, it may result in a sintered body with a shade whiter than natural teeth, whereas an excessive content may result in an increase of a yellow or red tint.

The mechanism by which the sintering aid (for example, magnesium oxide) enhances sintering density is believed to involve the exclusion of pores from the system by preventing the pores from being integrated into the grains, probably as a result of the sintering aid existing as a distinct phase at the grain boundaries, and inhibiting the growth and propagation of grain boundaries.

In applications requiring a sintered body with high purity, for example, 99.99 mass% or more, the content of the sintering aid in the alumina powder may be 10 to 100 ppm, or 20 to 50 ppm, in terms of the constituent element of the sintering aid (for example, in terms of a Mg element). The content of the sintering aid in an alumina pre-sintered body of the present invention and in the alumina composition mentioned later aligns with the content of the sintering aid in the alumina powder.

In view of increased linear light transmittance and translucency, the alumina particles contained in an alumina pre-sintered body of the present invention have an average primary particle diameter of 30 to 300 nm.

An average primary particle diameter of less than 30 nm is not preferable because it leads to an increased yellow tint in the sintered body. An average primary particle diameter of more than 300 nm is not preferable because it leads to an increase in grain size in the sintered body after sintering, resulting in an excessively large average crystal grain size, and a decrease in aesthetics and strength as dental materials.

An average primary particle diameter of 30 to 300 nm is preferable because the incorporation of smaller particles by larger particles becomes less likely in the sintering process of the pre-sintered body, reducing the occurrence of uneven sintering due to particle size differences, improving translucency and linear light transmittance.

The alumina particles have an average primary particle diameter of preferably 40 to 250 nm, more preferably 60 to 200 nm, even more preferably 80 to 150 nm.

The method of measurement of average primary particle diameter in the pre-sintered body is as described in the EXAMPLES section below.

A pre-sintered body of the present invention comprises a bluish colorant that imparts a bluish color after sintering (pigments, composite pigments; hereinafter, referred to as bluish colorant).

Examples of the pigments include at least one element selected from the group consisting of Ti, V, Cr, Mn, Fe, Co, Ni, Zn, Y, Zr, Sn, Sb, Bi, Ce, Sm, Eu, Gd, and Er.

Sintering the alumina pre-sintered body with a sintering aid containing magnesium results in an increase in b* value. It is therefore preferable that the bluish colorant be a bluish colorant that yields a negative b* value within alumina when used as a pigment to inhibit an increase of b* value.

Particularly preferably, the bluish colorant comprises a cobalt component (hereinafter, also referred to as "Co component").

The method of measurement of b* value is as described in the EXAMPLES section below.

The pigment component may be used alone, or two or more thereof may be used as a mixture. Concerning pigment coloration, it is preferable to use combinations of pigments that do not affect the a* value but result in a decrease in b* value.

In terms of pigment combinations, it is more preferable to combine, for example, a Co component with pigments that cause an increase in a* value because the Co component causes a simultaneous decrease in both b* value and a* value when added alone. For example, it is preferable to use Cr as an element that can inhibit a decrease in a* value due to Co when added in an amount equivalent to Co by mass. For example, the ratio of Co and Cr is preferably 0.8 to 1.25, more preferably 0.9 to 1.1.

The method of measurement of a* value is as described in the EXAMPLES section below.

The content of the Co component contained in an alumina pre-sintered body of the present invention is preferably 60 ppm or less, more preferably 50 ppm or less, even more preferably 25 ppm or less in terms of a cobalt element. With a Co content of 60 ppm or less, there is no excessive display of a bluish color, inhibiting yellowing in the sintered body after sintering, enhancing aesthetics at the incisal edge. Additionally, the Co component can integrate with the sintering aid to provide even superior translucency and linear light transmittance, enabling the reproduction of shades similar to natural teeth in dental applications.

In view of allowing the sintering aid to integrate with other constituents and enabling the sintered body to maintain high translucency and linear light transmittance and high strength even after sintering under atmospheric pressure, it is preferable in certain embodiments that the ratio (mass ratio) of the content of the Co component (in terms of a cobalt element) and the content of the sintering aid when it contains a Mg element (the content in terms of an element constituting the sintering aid (for example, in terms of a Mg element) be Mg:Co = 1,000:0.05 to 1,000:60, more preferably 1,000:0.1 to 1,000:55, even more preferably 1,000:0.5 to 1,000:50, particularly preferably 1,000:1 to 1,000:25.

The chemical state of the pigment is not particularly limited, as long as it exhibits coloration after the alumina is fired. The pigment may be a metal salt, an organometallic complex, a metal hydroxide, a metal oxide, or a combination of these in the form of oligomers or polymers. For example, in view of superior dispersibility and excellent uniform coloration, the cobalt component is preferably one derived from salts and/or complexes.

Specific examples of the bluish colorant include Co components such as bis(acetylacetonato)diaquacobalt(II), tris(acetylacetonato)cobalt(III), cobalt(II) amidosulfate hydrate, cobalt(II) benzoate, cis-tetraamminedichlorocobalt(III) chloride, pentaamminechlorocobalt(III) chloride, hexaamminecobalt(III) chloride, hexaamminecobalt(III) nitrate, ammonium diamminetetranitrocobaltate(III), triamminetrinitrocobalt(III), tetraamminedinitrocobalt(III) chloride, potassium diamminetetranitrocobaltate(III), cobalt(II) chloride hydrate (e.g., cobalt(II) chloride hexahydrate), cobalt(II) chloride, cobalt(III) chloride, cobalt(II) octanoate, cobalt(II) oleate, cobalt(II) perchlorate hydrate (cobalt(II) perchlorate hexahydrate), cobalt(II) fluoride hydrate (cobalt(II) fluoride dihydrate, cobalt(II) fluoride trihydrate, cobalt(II) fluoride tetrahydrate), dicobalt octacarbonyl (Co₂(CO)₈), cobalt(II) formate hydrate, cobalt(II) citrate hydrate, cobalt silicide, cobalt(II) acetate, cobalt(II) acetate hydrate (cobalt(II) acetate tetrahydrate), cobalt(II) oxide, cobalt(III) oxide, tricobalt tetraoxide (Co₃(CO)₄), potassium hexacyanocobaltate(III), cobalt(II) bromide, cobalt(II) bromide hydrate, cobalt(II) oxalate hydrate (e.g., cobalt(II) oxalate dihydrate, cobalt(II) oxalate tetrahydrate), cobalt resinate, cobalt(II) nitrate hydrate (cobalt(II) nitrate trihydrate, cobalt(II) nitrate hexahydrate), cobalt(II) metazirconate, cobalt(II) hydroxide, cobalt(III) hydroxide, cobalt(II) stearate, cobalt(II) selenate, cobalt(II) selenate hexahydrate, cobalt(II) tungstate tetrahydrate, cobalt(II) carbonate hydroxide, ammonium tetrakis(thiocyanato)cobaltate(II) hydrate, cobalt(II) thiocyanate, cobalt(II) metatitanate, potassium hexanitrocobaltate(III), sodium hexanitrocobaltate(III), hexaamminecobalt(III) sulfate, cobalt boride, cobalt(II) molybdate hydrate, cobalt(II) iodide hydrate, cobalt(II) laurate, cobalt(II) sulfide, cobalt(II) sulfate hydrate, cobalt(II) phosphide, and cobalt(II) phosphate hydrate.

Examples of composite pigments as bluish colorants include (Ni,Co,Fe)(Fe,Cr)₂O₄-ZrSiO₄, and (Co,Zn)Al₂O₄.

In view of dispersibility, preferred are cobalt(II) chloride hydrate, cobalt(II) perchlorate hydrate, cobalt(II) fluoride hydrate, cobalt(II) nitrate hydrate, cobalt(II) oxalate hydrate, and cobalt(II) acetate hydrate, more preferably cobalt(II) chloride hydrate. These Co components may be used alone, or two or more thereof may be used in appropriate combinations.

In view of aesthetics after alumina sintering, it is preferable that the pigment be uniformly dispersed in the pre-sintered body. An uneven presence of the pigment within the pre-sintered body results in localized coloration after sintering, leading to visually noticeable color inconsistencies.

The dispersion state of the pigment can be confirmed by compositional analysis of the pre-sintered body. Known methods can be employed for compositional analysis. For example, measurement may be conducted by scanning or transmission electron microscopy (SEM or TEM) and energy dispersive X-ray analysis or wavelength dispersive X-ray analysis (EDX or WDX), or field emission electron probe microanalysis (FE-EPMA).

For the measurement of the dispersion state of the pigment, the alumina pre-sintered body containing the pigment may be analyzed under the following conditions, using, for example, a field-emission scanning electron microscope (FE-SEM Reglus 8220 manufactured by Hitachi High-Tech Corporation) and an energy-dispersive X-ray analyzer (Aztec Energy X-Max 50 manufactured by Oxford Instruments).
Measurement magnification: 20,000×
Analysis mode: line analysis
Acceleration voltage: 5 kV
Working distance: 15 mm ± 1 mm
X-ray extraction angle: 30 degrees
Dead time: 7%
Measurement time: 100 seconds

In the region where alumina is detected by line analysis, the physical distance representing the concentration difference of the elements constituting the pigment can be determined by measuring the physical distance between a single maximum value and the surrounding minimum values, and averaging the physical distances between adjacent maximum and minimum values in the waveform representing the detected amounts obtained through line analysis for the constituent elements of the pigment. Preferably, the field is adjusted in different positions so that there are at least 20 connected fields, with a distance of at least 50 µm within the connected field in line analysis. Preferably, analysis is conducted by measuring a minimum of twenty locations. Preferably, the physical distance represents the average value from the waveforms obtained by line analysis.

The physical distance representing the concentration difference of the elements constituting the pigment is preferably within 50 µm because this ensures that the concentration difference of the pigment, exhibiting color upon firing, remains visually imperceptible. The concentration difference of the elements constituting the pigment is more preferably within 30 µm, even more preferably within 10 µm.

Instead of measuring the physical distance representing the concentration difference of the elements constituting the pigment, it is possible to employ Fourier transformation for the frequency analysis of the waveform of the detected amounts obtained through line analysis for the constituent elements of the pigment in the region where alumina is detected by line analysis. The period determined from the frequency corresponding to the peak top in the Fourier spectrum is preferably within 50 µm. A period exceeding 50 µm is not preferable because it raises the possibility of visual perceptibility of the concentration difference of the pigment exhibiting color upon firing. The concentration difference of the elements constituting the pigment is more preferably within 30 µm, even more preferably within 10 µm.

Preferably, the pigment has high dispersibility because it helps to reduce the decrease in the linear light transmittance and strength of the sintered body. This can be assessed by the physical distance determined from the waveform of line analysis, or the period of Fourier analysis, using these as indicators.

Preferably, alumina represents the main component in a pre-sintered body of the present invention because it leads to enhanced aesthetics in the sintered body as a dental material, in addition to improving chemical stability. Here, the main component refers to the component with the highest content. The content of the main component may be, for example, 50 mass% or more, 70 mass% or more, 80 mass% or more, or 90 mass% or more.

The alumina particles contained in a pre-sintered body of the present invention are more preferably α-alumina with a purity of 99.5% or more because of its low impurity content, and the ability to reduce the impurity-related formation of a glass phase at crystal grain boundaries, preventing the coarsening of crystal grains (grains), and inhibiting a decrease of aesthetic qualities in the sintered body as a dental material.

Using α-phase aluminum oxide (α-alumina) as the starting raw material is preferable because, with its high corrosiveness and high stability at elevated temperatures, it enables uniform control of the pre-sintered body, and the maintenance of high translucency and linear light transmittance after firing, in addition to enabling densification by preventing coarsening of grain size in the crystalline structure within the sintered body.

Considering these factors, it is particularly preferable that the alumina particles contained in a pre-sintered body of the present invention comprise α-alumina with a purity of 99.5% or more.

The alumina raw material can be acquired using methods, for example, such as the alkoxide method, modified Bayer method, ammonium alum pyrolysis method, and ammonium dawsonite pyrolysis method, preferably the alkoxide method. The alkoxide method allows for enhancement of purity in a powder of alumina raw material, making it easier to achieve a uniform particle size distribution. As a specific example, aluminum hydroxide obtained through hydrolysis of purified aluminum alkoxide is fired in air at 1,100°C or higher temperatures.

Examples of the alumina raw material include α-alumina with a purity 99.99% or more, for example, such as the AKP grade (α-alumina) and the NXA grade (e.g., NXA-100, NXA-150) manufactured by Sumitomo Chemical Co., Ltd. (These are all ultrafine α-alumina.)

In view of enhancing strength after sintering, and providing high aesthetic qualities in particular, it is preferable that an alumina pre-sintered body of the present invention comprise the sintering aid.

The content of the sintering aid or pigment in a pre-sintered body of the present invention and in a sintered body thereof can be measured by, for example, inductively coupled plasma (ICP) emission spectral analysis, X-ray fluorescence analysis (XRF), scanning or transmission electron microscopy (SEM or TEM), energy dispersive X-ray analysis or wavelength dispersive X-ray analysis (EDX or WDX), or field emission electron probe microanalysis (FE-EPMA).

A certain embodiment is, for example, an alumina pre-sintered body for dental use that shows a linear light transmittance of 0.8% or more as a sintered body of 1.0 mm thickness fired under atmospheric pressure without a hot isostatic pressing process. The linear light transmittance shares the same measurement method and preferred ranges as the linear light transmittance of the alumina sintered body described later.

The linear light transmittance of a sintered body of 1.0 mm thickness after firing the alumina pre-sintered body for dental use under atmospheric pressure without a hot isostatic pressing process is preferably 0.8% or more, more preferably 1% or more, even more preferably 1.1% or more.

Another certain embodiment is, for example, an alumina pre-sintered body for dental use that shows a b* value of -8.0 to 14.2 as a sintered body of 1.2 mm thickness fired under atmospheric pressure without a hot isostatic pressing process. The b* value is preferably -8.0 to 14.2, more preferably -7.0 to 14.0, even more preferably -6.0 to 13.8.

In certain preferred embodiments, the b* value is preferably 0 to 14.0, more preferably 0.1 to 13.9, even more preferably 0.2 to 13.8.

The measurement method of b* value is the same as that for the b* value of the alumina sintered body described later.

The following describes a composition (hereinafter, also referred to as "alumina composition") for producing a pre-sintered body of the present invention. The alumina composition comprises alumina, a bluish colorant, and a sintering aid. Examples of the alumina, bluish colorant, and sintering aid include those exemplified for the alumina pre-sintered body.

The alumina composition becomes a precursor of an alumina pre-sintered body of the present invention.

The alumina composition and molded body refer to a state before firing, and involve no necking between alumina particles.

The content of alumina, bluish colorants, and sintering aids in an alumina composition of the present invention is calculated from their content in a predetermined alumina pre-sintered body, and is the same for the alumina composition and alumina pre-sintered body.

An alumina composition of the present invention is not limited to particular forms, and may have various forms, including a powder, a fluid with a powder added to solvent, and a molded body of a powder formed into a predetermined shape. When an alumina composition of the present invention has a powder form, it may be a cluster of granules. The granules are formed by the aggregation of primary particles.

In this specification, "primary particle" refers to a bulk representing the smallest unit. For example, "primary particle" refers to particles that appear spherical under an electron microscope (for example, a scanning electron microscope). The primary particles include alumina particles. When employing particulate sintering aids, the primary particles include particles of sintering aid, as well as alumina particles.

Preferably, the particles constituting the granules formed of the alumina composition are predominantly primary particles. Aggregates of primary particles are referred to as secondary particles. Preferably, the number of primary particles is greater than the number of secondary particles in visual confirmation through, for example, an electron microscope. Secondary particles are typically irregular in shape, and an increase in their quantity leads to sparse density during the press molding process described later, resulting in a decrease of aesthetics qualities due to the coarse density after sintering.

The particle diameter of primary particles within the particles constituting the granules formed of the alumina composition influences the extent of necking during pre-sintering, and affects the hardness of the pre-sintered body.

Particles with an average primary particle diameter of less than 30 nm are not preferable because it results in strong necking, leading to a decrease in the surface area of the primary particles contained in the pre-sintered body, and a hardness increase during the machining process described below. Particles with an average primary particle diameter of more than 300 nm are not preferable because the incorporation of smaller particles in the particle size distribution becomes more likely, causing localized necking due to particle size differences, thereby increasing the occurrence of coarse density.

The average primary particle diameter is preferably 30 to 300 nm, more preferably 40 to 250 nm, even more preferably 60 to 200 nm.

The primary particles within the particles constituting the granules formed of the alumina composition may be a combination of two types of alumina particles having different average primary particle diameters. For example, when the NXA is used, an example is a combination of NXA-100 (ultrafine α-alumina manufactured by Sumitomo Chemical Co., Ltd.) and NXA-150 (ultrafine α-alumina manufactured by Sumitomo Chemical Co., Ltd.).

The particles constituting the granules formed of the alumina composition has a BET specific surface area of preferably 5 m²/g or more, more preferably 7.5 m²/g or more, even more preferably 8 m²/g or more, as measured in compliance with JIS Z 8830:2013.

With a BET specific surface area of 5 m²/g or more, it is easier to lower the sinterable temperature. This facilitates easier sintering, or makes it easier to inhibit the reduction in translucency due to the opacification occurring in the sintered body after sintering.

The BET specific surface area is preferably 25 m²/g or less, more preferably 22 m²/g or less, even more preferably 18 m²/g or less.

With a BET specific surface area of 25 m²/g or less, the average primary particle diameter is not excessively small, reducing the occurrence of coarse density in the pre-sintered body, providing even superior aesthetics after sintering.

With a BET specific surface area of 25 m²/g or less, the pre-sintered body can be prevented from becoming overly hard. This facilitates a reduction in the amount of wear in tools (hereinafter, also referred to as "amount of tool wear") and/or chipping rate, or reduces the occurrence of coarse density by ensuring sufficient necking, facilitating a reduction in chipping rate.

In this specification, "BET specific surface area" refers to the specific surface area measured without distinguishing between primary particles and secondary particles.

Concerning the BET specific surface area of a pre-sintered body of the present invention and the BET specific surface area of the composition described above, the extent of necking is preferably such that the numerical difference obtained by subtracting the BET specific surface area of the pre-sintered body from the BET specific surface area of the composition is within 10 m²/g. This makes it possible to desirably maintain cutting and grinding properties.

An alumina composition of the present invention may employ a granular form in 50% or more, preferably 70% or more, more preferably 80% or more, even more preferably 90% or more of the alumina in the alumina composition.

When an alumina composition of the present invention does not employ a granular form, the alumina particles constituting the powder may have the average particle diameter and the BET specific surface area noted above.

The average particle diameter of granules in an alumina composition of the present invention (secondary particle diameter; hereinafter, also referred to as "average granule diameter") is preferably 10 µm or more, more preferably 12 µm or more, even more preferably 14 µm or more.

When the average granule diameter is less than 10 µm, there is a risk of trapping air in the process of placing the granules in a die, leading to inadequate degassing during molding. This may result in the inability to fabricate a uniform and dense molded body. Additionally, there is a risk of granules being ejected through gaps during molding, potentially resulting in a molded body that does not meet the predetermined quantitative requirements. The average granule diameter is preferably 200 µm or less, more preferably 190 µm or less, even more preferably 180 µm or less, particularly preferably 150 µm or less, most preferably 100 µm or less.

When the average granule diameter exceeds 200 µm, there is an increased likelihood of cavity formation within the granules. Additionally, voids are more likely to occur when placing the granules in a die. These phenomena pose a risk of inadequate degassing during molding, potentially resulting in the inability to fabricate a dense molded body. There is also a risk of increased shrinkage during molding, potentially leading to the inability to fabricate a molded body of the desired size.

It is preferable that 50% or more of the alumina in the alumina composition constitute granules. Preferably, the average granule diameter is measured using a method that does not disrupt the granules. The average granule diameter can be measured using a method, for example, such as dry sieving or wet sieving.

Measurement by dry sieving can be conducted following the test sieving described in JIS Z 8815:1994. Both manual sieving and mechanical sieving are applicable; however, mechanical sieving is preferred.

For sieving, the test sieve described in JIS Z 8801-1:2019 can be used.

Ro-Tap sieve shakers or sonic sieving analyzers may be used as measurement instruments for sieving, for example. Examples of the Ro-Tap sieve shakers include RPS-105M manufactured by Seishin Enterprise Co., Ltd. Examples of the sonic sieving analyzers include Robot Sifter RPS-01 and Robot Sifter RPS-02 manufactured by Seishin Enterprise Co., Ltd.

It is preferable that granules in an alumina composition of the present invention have high sphericity. By increasing the sphericity of granules, it is possible to promote mixing at the interface between layers when stacking alumina powders of different compositions. With increased sphericity, it is also possible to increase the packing density of when an alumina powder is filled into a mold to prepare a molded body, even when the average particle diameter is the same. The strength and translucency of the sintered body can increase by increasing the packing density, which is a density of a molded body formed into a specific shape under pressure after alumina granules are filled into a specific mold (such as a die). It is also possible to more easily fill granules into angular portions when the mold has angular portions.

The sphericity of granules in an alumina composition of the present invention can be represented by parameters, for example, such as light bulk density, and heavy bulk density.

In view of increasing granule flow (ease of loading) to reduce coarseness in the density of the molded body obtained, an alumina composition of the present invention has a light bulk density of preferably 0.6 g/cm³ or more, more preferably 0.7 g/cm³ or more, even more preferably 0.8 g/cm³ or more, particularly preferably 0.9 g/cm³ or more.

The light bulk density can be measured in compliance with JIS R 9301-2-3:1999.

In view of increasing granule flow (ease of loading) to reduce coarseness in the density of the molded body obtained, an alumina composition of the present invention has a heavy bulk density of preferably 0.8 g/cm³ or more, more preferably 0.9 g/cm³ or more, even more preferably 1.0 g/cm³ or more.

The heavy bulk density can be measured in compliance with JIS R 9301-2-3:1999.

An alumina composition of the present invention preferably comprises a binder.

The binder may be, for example, an organic binder. Examples of the organic binder include common binders such as acrylic binders, acrylate binders, paraffinic binders, fatty acid binders, and polyvinyl alcohol binders. Preferred among these organic binders is one having a carboxyl group in the molecular chain, or a derivative of carboxylic acids, more preferably an acrylic binder, even more preferably a polyacrylate having water solubility. The polyacrylate may be a product of copolymerization of acrylic acid or methacrylic acid with maleic acid, and may contain sulfonic acid. Examples of the cations of the salt include sodium and ammonium.

Depending on the content of the binder in an alumina composition of the present invention, it is possible to adjust the distance between primary particles in the alumina composition, and the cumulative distribution of pores, making it easier to increase or decrease the Vickers hardness or the strength of the pre-sintered body.

The binder content is preferably 1.2 to 2.8 mass%, more preferably 1.5 to 2.5 mass%, even more preferably 1.8 to 2.2 mass% in the whole alumina composition. When the binder content is 1.2 mass% or more in the whole alumina composition, the pre-sintered body does not become overly strong, eliminating the risk of hardening when removing the cut workpiece.

When the binder content is 2.8 mass% or less, the strength of the pre-sintered body does not overly decrease, reducing the possibility of the workpiece detaching during cutting, in addition to facilitating a reduction in chipping rate.

In addition to the bluish colorant, an alumina composition of the present invention may optionally comprise additives other than sintering aids (additives excluding CeO₂, ZrO₂, and Y₂O₃), such as colorants (including pigments, composite pigments, and fluorescent agents, excluding bluish colorants), titanium oxide (TiO₂), silica (SiO₂), dispersants, and antifoaming agents. These components may be used alone, or two or more thereof may be used as a mixture.

Examples of the pigments include oxides of at least one element selected from the group consisting of Ti, V, Cr, Mn, Fe, Ni, Zn, Y, Zr, Sn, Sb, Bi, Ce, Sm, Eu, Gd, and Er, excluding the bluish colorant.

Examples of the composite pigments include (Zr,V)O₂, and Fe(Fe,Cr)₂O₄.

Examples of the fluorescent agents include Y₂SiO₅:Ce, Y₂SiO₅:Tb, (Y,Gd,Eu)BO₃, Y₂O₃:Eu, YAG:Ce, ZnGa₂O₄:Zn, and BaMgAl₁₀O₁₇:Eu.

The additives may be added during mixing or pulverization, or may be added after pulverization.

A certain embodiment is, for example, a dental mill blank comprising a portion with the alumina pre-sintered body. By comprising a portion with the alumina pre-sintered body, the dental mill blank can exhibit the high level of aesthetics required for the incisal edge of incisors or canines, without the need for complementation by other materials (for example, porcelain), due to the high translucency and high linear light transmittance of the alumina pre-sintered body exhibited upon sintering. The location where such a portion is situated within the dental mill blank can be adjusted during processing, and is not specified.

Another certain embodiment is, for example, a dental mill blank in which said portion is a portion with the highest translucency. With said portion representing a portion with the highest translucency, the dental mill blank can exhibit particularly high aesthetics when this portion is processed into the incisal region of incisors or canines. The location where such a portion is situated within the dental mill blank can be adjusted during processing, and is not specified.

A certain embodiment is, for example, a method for producing an alumina pre-sintered body for dental use, comprising the step of allowing a bluish colorant to uniformly adhere to alumina particles,
wherein the alumina pre-sintered body for dental use comprises alumina particles with an average primary particle diameter of 30 to 300 nm, a sintering aid, and a bluish colorant, and
the content of the sintering aid is 10 to 5,000 ppm.

The step of allowing a bluish colorant (for example, a cobalt component) to uniformly adhere to alumina particles may comprise the step of dispersing α-alumina particles in a dispersion medium with the bluish colorant dissolved in the dispersion medium. For example, the method may comprise dispersing α-alumina particles in a dispersion medium with the bluish colorant (for example, a cobalt component) dissolved in the dispersion medium. The dispersion medium is not particularly limited, and may be, for example, an organic solvent (e.g., alcohol-based solvents such as methanol and ethanol). The dispersion medium may be used alone, or two or more thereof may be used in combination. By allowing the bluish colorant to uniformly adhere to alumina particles, the alumina pre-sintered body for dental use can exhibit particularly high aesthetics when processed into the incisal region of incisors or canines.

The method for producing an alumina pre-sintered body for dental use may comprise the step of obtaining a molded body by pressure molding of an alumina composition.

An example of an alumina pre-sintered body producing method comprises the steps of:
producing an alumina composition comprising alumina particles, a bluish colorant, and a sintering aid; and
firing (pre-sintering) the alumina composition (for example, a molded body) to obtain an alumina pre-sintered body in which the alumina particles within the pre-sintered body have an average primary particle diameter is 30 to 300 nm, and the content of the sintering aid is 10 to 5,000 ppm.

The process of producing an alumina composition of the present invention is described first.

First, alumina and a sintering aid are mixed in predetermined proportions to prepare a mixture (mixing step). For example, when the sintering aid is magnesium chloride, alumina and magnesium chloride may be mixed at a mixing ratio that provides the foregoing contents. The mixing process may be dry mixing or wet mixing. The alumina composition may be pulverized (preferably, crushed) until the foregoing average particle diameter is achieved (pulverization step).

The mixing and pulverization may be performed in a single step. Pulverization can be performed by using, for example, a ball mill or bead mill after dispersing the composition and binder in a solvent such as water or alcohol (dispersing step). The composition is pulverized (preferably, crushed) to achieve an average primary particle diameter of, for example, 0.05 µm to 0.3 µm in the composition. For particle size adjustment, the composition may optionally be subjected to other processes (classification, elutriation).

The average primary particle diameter can be measured by laser diffraction/scattering particle size distribution analysis. For example, the average primary particle diameter can be measured by volume with ultrasonic waves being applied after a slurry diluted with water is subjected to 30 minutes of ultrasonication, using a laser diffraction/scattering particle size distribution analyzer (manufactured by Horiba Ltd. under the trade name Partica LA-950).

After the mixing and/or pulverization step, the mixture may be dried by spray drying with a spray dryer or the like to achieve the aforementioned granular form in the alumina composition (drying step).

In the pulverization step, the average particle diameter of the alumina composition is preferably 0.3 µm or less, more preferably 0.25 µm or less, even more preferably 0.2 µm or less, particularly preferably 0.15 µm or less. When the average particle diameter of the alumina composition is less than 0.15 µm, the sintered body can exhibit enhanced aesthetics by retaining the small crystal grain size. These average particle diameters are also preferable in terms of improved machinability.

Alumina and sintering aids may be separately prepared. For example, it is possible to independently provide separate preparation steps (for example, production steps) for alumina and sintering aids, instead of simultaneously precipitating alumina and sintering aids (in the same step). In this way, the aforementioned α-alumina can be obtained with high purity and a small primary particle diameter.

Typically, in the production of alumina compositions, sintering aids may be reacted with alumina through heat treatment, and the resulting product may be used for the pulverization and drying steps.

This enables the production of granules formed of the alumina composition, which serves as the raw material of alumina pre-sintered bodies.

The granule or powder can be formed into a molded body by applying an external force. The molding method is not limited to specific methods, and a suitable method may be appropriately selected according to intended use. For example, molding may be achieved by press molding, injection molding, stereolithography, slip casting, gel casting, filtration-casting, or casting. Molding may be performed in multiple stages. For example, the alumina composition may be subjected to a CIP process after press molding, or press molding and CIP molding may be repeated.

Examples of the press molding method include a uniaxial pressing process (hereinafter, also referred to as "uniaxial pressing"), a biaxial pressing process, and a CIP (Cold Isostatic Pressing) process. These may be performed in combination, as needed.

A molded body of the present invention may have a disc shape, a cuboidal shape, or a shape of a dental product (for example, a crown shape).

A certain embodiment is, for example, an alumina pre-sintered body producing method in which the press molding is uniaxial pressing, and the surface pressure of uniaxial pressing is 5 MPa or more. The press molding may yield, for example, a columnar molded body packed by uniaxially pressing alumina granules filled into a die. The molded body can increase its density with higher surface pressure during press molding. Conversely, a hard alumina pre-sintered body results when the density of the molded body is excessively high. The surface pressure in press molding is preferably 5 to 600 MPa, more preferably 10 to 400 MPa, even more preferably 15 to 200 MPa. When the surface pressure of pressing is 5 MPa or more, the molded body exhibits superior shape retention. With a surface pressure of 600 MPa or less, it is possible to prevent excessive density increase in the molded body, making it easier to avoid hardening.

A molded body of the present invention encompasses molded bodies compacted by a high-temperature pressing process such as a CIP (Cold Isostatic Pressing) process. In view of the above, the hydraulic pressure is preferably 50 to 1,000 MPa, more preferably 100 to 600 MPa, even more preferably 150 to 300 MPa.

An alumina pre-sintered body according to the present invention becomes a precursor (intermediate product) of an alumina sintered body according to the present invention.

The pre-sintered body includes those that have been subjected to a molding process. For example, an alumina pre-sintered body according to the present invention includes dental products (for example, crown-shaped prostheses) processed from pre-sintered alumina discs with the CAD/CAM (Computer-Aided Design/Computer-Aided Manufacturing) system.

The content of the alumina and sintering aids in an alumina pre-sintered body of the present invention is the same as that in the alumina composition to be fabricated into an alumina pre-sintered body. In view of the strength and translucency of sintered bodies fabricated from an alumina pre-sintered body of the present invention, the stabilizing agent is preferably a magnesium compound for its uniform dispersibility.

The firing temperature (hereinafter, also referred to as "pre-sintering temperature") in the step of firing under atmospheric pressure in the pre-sintered body producing method (pre-sintering step) affects the Vickers hardness, or the strength of the pre-sintered body.

The pre-sintered body undergoes changes in cumulative pore distribution and hardness with the pre-sintering temperature, leading to changes in the amount of tool wear and/or chipping rate.

In view of this, the pre-sintering temperature (highest pre-sintering temperature) in an alumina pre-sintered body producing method of the present invention is preferably 600°C to 1,200°C, more preferably 650°C to 1,100°C, even more preferably 700°C to 1,000°C.

When the pre-sintering temperature is 600°C or more, the pillar (support or sprue) that connects a section of the pre-sintered body to the workpiece while it is being cut or ground by machining the pre-sintered body can remain unbroken, preventing the workpiece from being detached during the cutting or grinding process. Additionally, the Vickers hardness can be adjusted within the desired range to reduce an increase in chipping rate. When the pre-sintering temperature is 1,200°C or less, there is no excessive neck formation, preventing the workpiece from becoming too hard. This facilitates a quicker separation of the workpiece from the frame securing the workpiece. Additionally, an increase in chipping rate can be reduced as tool wear remains low, allowing for an easy separation of the workpiece from the pillar.

It is preferable to maintain the highest pre-sintering temperature for a certain time period because it confines the hardness of the pre-sintered body within the preferred range, and may lead to a reduction in chipping rate. The pre-sintering conditions depend on the average primary particle diameter of the pre-sintered body, and the density of the pre-sintered body. The holding time at the highest pre-sintering temperature is preferably 30 minutes to 6 hours. Preferably, the rate of temperature increase to the highest pre-sintering temperature, and the rate of temperature decrease from the highest pre-sintering temperature are 300°C/min or less.

An alumina pre-sintered body of the present invention can be machined to fabricate a workpiece. The processing method is not limited to specific methods, and a suitable method may be appropriately selected according to intended use. For example, an alumina disc (pre-sintered body) can be milled or ground into a shape of a dental product (for example, a crown-shaped prosthesis) with a CAD/CAM system to fabricate a workpiece.

The surface smoothness of the workpiece can be improved using tools such as polishers (for example, Pearl Surface^{®} manufactured by Kuraray Noritake Dental Inc.).

In a pre-sintered body of the present invention, the relative density can be controlled by the method of production described later.

A relative density of less than 43% means that the proportion of pores inside the pre-sintered body is high, leading to increased chipping due to sparsity in the relative density inside the pre-sintered body. This sparsity results in uneven shrinkage rates during sintering, causing some deformation in the sintered body, increasing the need for reworking. In view of the aesthetic qualities of the sintered body, it is not preferable to have sparsity in relative density. This is because, though the presence of sparsity may improve processability such as cutting and grinding properties, a high proportion of pores within the pre-sintered body means that the distance between particles is large, resulting in the inability to completely release voids out of the sintered body during the sintering process, leading to a decrease in the aesthetic qualities of the sintered body.

A relative density of 43% to 63% is preferable because it provides a good overall balance between particles and pores, enabling a reduction in the amount of tool wear and/or chipping rate, and the maintenance of high aesthetic qualities in the sintered body. The relative density is preferably 43% to 63%, more preferably 50% to 55%.

The relative density of the pre-sintered body can be calculated from the porosity of the pre-sintered body. Specifically, a mercury porosimeter can be used for the measurement and calculation of relative density. The mercury porosimeter is preferably one capable of applying a mercury pressure of 15 to 30,000 psia, preferably 0.5 to 60,000 psia.

In view of reducing measurement errors, the preferred pressure resolution is 0.1 psia or higher.

Examples of the mercury porosimeter include AutoPore^{®} IV 9500, manufactured by Micromeritics (USA).

The density of the pre-sintered body is defined as the density of a pre-sintered body obtained after granules resulting from drying of raw materials are filled into a specific mold (such as a die) and molded into a specific shape under pressure, and the resulting molded body is subsequently heated to remove binders at a temperature that allows for removal of binders, and heated again at a temperature that allows for moderate formation of a solid solution with yttria, and a moderate level of necking. The temperature for the removal of binders is not particularly limited, as long as it allows for removal of binders, and may be 150 to 500°C. The temperature at which moderate neck formation occurs is preferably 700 to 1,200°C.

A pre-sintered body of the present invention exhibits a change in BET specific surface area with the average primary particle diameter, the state of necking, and density. The BET specific surface area can be measured in compliance with JIS Z 8830:2013. For the measurement of BET specific surface area, commercially available products can be used, such as a full automatic specific surface area analyzer (manufactured by Mountech Co., Ltd. under the trade name Macsorb^{®} HM Model-1200, BET flow method (single point/multi point)).

In view of decreasing the amount of tool wear and chipping rate, a pre-sintered body of the present invention has a BET specific surface area of preferably 5 m²/g or more, more preferably 7.5 m²/g or more, even more preferably 8 m²/g or more.

With a BET specific surface area of 5 m²/g or more, the average primary particle diameter is not excessively large, allowing for the reduction of an increase in chipping rate, or the prevention of excessive necking, thereby reducing an increase in the amount of tool wear.

The BET specific surface area is preferably 25 m²/g or less, more preferably 22 m²/g or less, even more preferably 18 m²/g or less.

With a BET specific surface area of 25 m²/g or less, the average primary particle diameter is not excessively small, preventing the pre-sintered body from becoming overly hard. This facilitates a reduction in the amount of tool wear and/or chipping rate, or allows for a reduction of density coarseness by ensuring sufficient necking, making it easier to reduce the chipping rate.

Concerning the processability of a pre-sintered body of the present invention, the processability is also influenced by the strength of the pre-sintered body. The strength of a pre-sintered body according to the present invention can be evaluated by, for example, measuring the flexural strength of the pre-sintered body.

The three-point flexural strength of a pre-sintered body according to the present invention can be measured in compliance with JIS R 1601:2008.

In order to ensure strength that enables machining, the pre-sintered body has a three-point flexural strength of preferably 10 MPa or more, more preferably 18 MPa or more, even more preferably 20 MPa or more. When the pre-sintered body has a three-point flexural strength of less than 10 MPa, the pillar (support or sprue) becomes more likely to break during the cutting or grinding process, increasing the possibility of the workpiece separating from the pre-sintered body before it becomes a cut or ground workpiece.

For easy machining, the pre-sintered body has a three-point flexural strength of preferably 50 MPa or less, more preferably 45 MPa or less, even more preferably 40 MPa or less, particularly preferably 35 MPa or less.

In view of reducing the amount of tool wear or chipping rate, and enabling easy separation of the cut or ground workpiece from the fixture frame for quick removal while reducing tool wear, a pre-sintered body of the present invention has a Vickers hardness of 350 HV 5/30 or less, preferably 300 HV 5/30 or less, more preferably 100 HV 5/30 or less. A Vickers hardness of less than 30HV 5/30 leads to an increase in the frequency of chipping, whereas the amount of tool wear increases with a Vickers hardness of more than 135HV 5/30.

Here, "HV 5/30" means a Vickers hardness under the application of a load (test force) of 5 kgf for a duration of 30 seconds.

A pre-sintered body of the present invention can have a reduced probability of chipping with the Vickers hardness falling in the foregoing predetermined ranges.

The Vickers hardness in the present invention is measured using a method in compliance with JIS Z 2244:2020, as will be described later in detail in the EXAMPLES section.

The Vickers hardness in a pre-sintered body of the present invention becomes more easily achievable with factors such as the average primary particle diameter of particles contained in the pre-sintered body, the relative density of the pre-sintered body, and the strength of the pre-sintered body due to the state of particle necking.

The production methods of pre-sintered bodies and compositions are important to achieve these factors. This involves a composition producing method that essentially does not involve the firing step, the binder content in producing the composition, the surface pressure during the fabrication of the molded body, and the pre-sintering temperature in the production of the pre-sintered body. These are described below.

The processing machine used for machining a pre-sintered body of the present invention is not particularly limited. For example, milling or grinding machines such as desktop processing machines and large machining centers (general-purpose processing machine) may be used, depending on the workpiece to be processed. Examples of such milling machines include tabletop processing machines, for example, such as DWX-50, DWX-4, DWX-4W, DWX-52D, and DWX-52DCi (manufactured by Roland DG Corporation).

The tools employed in the processing machines for machining a pre-sintered body of the present invention are not particularly limited. Milling burs and grinding burs recommended by processing machine suppliers can be suitably used. A KATANA^{®} drill represents an example of milling burs used for milling machines.

The lifetime of tools used in machining equipment is contingent on usage conditions. For example, when cutting or grinding the pre-sintered body, wearing of the drill blade (tool wear) may lead to problems such as the processed surface of the pre-sintered body fracturing into small pieces (chipping) or large pieces. This results in productivity-related issues such as a time-consuming rework of the machining process. Chipping is particularly prominent in traditional pre-sintered bodies.

To assess tool life, torque on the machine side can be monitored, and a specific torque value may be established as the upper threshold limit, serving as an indicator to determine tool life (an indicator to decide when the tool should be replaced). Alternatively, the processing time may serve as the upper threshold limit. Verification of tool life may involve, for example, measuring the wear width of the blade on the milling bur used in the cutting or grinding machine. In the case of a KATANA^{®} drill for example, the tool life (replacement time) can be identified when the wear width reaches 0.21 mm or more. The wear width of the blade is preferably within 0.2 mm, more preferably within 0.15 mm, even more preferably within 0.1 mm.

An alumina sintered body of the present invention can be fabricated by sintering an alumina pre-sintered body of the present invention, or a cut or ground workpiece thereof, at a temperature that sinters the alumina particles (sintering step).

The sinterable temperature (for example, highest sintering temperature) is preferably 1,300°C or more, and may vary with the average primary particle diameter. When the average primary particle diameter is approximately 100 nm, the sinterable temperature (for example, highest sintering temperature) is, for example, preferably 1,300°C or more, more preferably 1,350°C or more, even more preferably 1,375°C or more.

The sinterable temperature is, for example, preferably 1,500°C or less, more preferably 1,450°C or less. The rate of temperature increase and the rate of temperature decrease are preferably 300°C/min or less.

In the sintering step, the holding time at the sinterable temperature (for example, highest sintering temperature) is preferably less than 120 minutes, more preferably 90 minutes or less, even more preferably 75 minutes or less, yet more preferably 60 minutes or less, particularly preferably 45 minutes or less, most preferably 30 minutes or less. The holding time is preferably 1 minute or more, more preferably 3 minutes or more, even more preferably 5 minutes or more.

An alumina composition and alumina pre-sintered body of the present invention enable a shorter sintering process to be employed for the fabrication of a sintered body, without causing a decrease in the translucency and strength of the alumina sintered body fabricated. Particularly, it is possible to shorten the holding time at the highest sintering temperature for the fabrication of a sintered body (short sintering). This enhances production efficiency, and when an alumina pre-sintered body of the present invention is applied to dental products, the patient can experience less time-related stress because it takes a shorter time before a treatment can be started with a dental product after its dimensions are determined and the product is cut or ground for the treatment. It is also possible to reduce the energy cost.

In the sintering step, the holding time at the sinterable temperature (for example, highest sintering temperature) may be, for example, 25 minutes or less, 20 minutes or less, or 15 minutes or less.

Preferably, the rate of temperature increase to the highest sintering temperature, and the rate of temperature decrease from the highest sintering temperature in the sintering process are set so as to reduce the time required for the sintering process. For example, the rate of temperature increase may be set so that the highest sintering temperature is reached in the shortest possible time, depending on the capabilities of the furnace.

The rate of temperature increase to the highest sintering temperature may be, for example, 10°C/min or more, 50°C/min or more, 100°C/min or more, 120°C/min or more, 150°C/min or more, or 200°C/min or more. Preferably, the rate of temperature decrease from the highest sintering temperature is set to a rate that does not cause deformation due to shrinkage rate differences, or defects such as cracks, in the sintered body.

For example, the sintered body may be allowed to cool at room temperature after the heating is finished.

The following describes an alumina sintered body obtained by sintering an alumina pre-sintered body of the present invention or a workpiece thereof. Here, "alumina sintered body" refers to, for example, a state where alumina particles (powder) have sintered. The alumina sintered body has a relative density of preferably 99.5% or more.

The relative density in the sintered body can be calculated as a ratio of the actual density, measured by the Archimedes method, with respect to the theoretical density. By "relative density", it means a value obtained by theoretically dividing density d1 by density d2, where d1 is the density of a sintered body after high-temperature firing of a molded body prepared by filling granules into a specific mold, and pressing the granules into a specific shape, and d2 is the density of alumina (with no internal voids).

An alumina sintered body of the present invention encompasses not only sintered bodies of molded alumina particles sintered under ordinary pressure and no applied pressure, but also sintered bodies compacted by a high-temperature pressing process such as a HIP process.

A greater relative density in an alumina sintered body of the present invention leads to fewer internal voids and less scattering of light. Because this results in increased translucency (ΔL), total transmittance, and linear light transmittance, and in view of improved aesthetics and strength in an alumina sintered body of the present invention, it is preferable for an alumina sintered body of the present invention to exhibit higher relative density. An alumina sintered body of the present invention has a relative density of, for example, preferably higher than 95%, more preferably 98% or more, even more preferably 99.5% or more. Most preferably, an alumina sintered body of the present invention is essentially void free.

A smaller average crystal grain size in an alumina sintered body of the present invention is preferable because it leads to increased linear light transmittance in the sintered body.

The average crystal grain size in the alumina sintered body is preferably 0.3 to 8.0 µm. The average crystal grain size is more preferably 5 µm or less, even more preferably 3 µm or less, yet more preferably 2 µm or less, particularly preferably 1 µm or less.

In an alumina sintered body of the present invention, an average crystal grain size of 0.3 to 8.0 µm is preferable because it also leads to increased strength, translucency (ΔL), and/or total transmittance.

The average crystal grain size of the alumina sintered body can be measured using the method described in the EXAMPLES section below.

An alumina sintered body of the present invention has a linear light transmittance of preferably 0.8% or more, more preferably 1% or more, even more preferably 1.1% or more at a thickness of 1.0 mm.

When the linear light transmittance of the alumina sintered body is less than 0.8% at a thickness of 1.0 mm, it may lead to the inability to provide the translucency (transmissivity for linear light) required for the incisal region of dental prostheses.

The method of measurement of the linear light transmittance of the alumina sintered body is as described in the EXAMPLES section below.

An alumina sintered body of the present invention has a linear light transmittance of preferably 20% or less, more preferably 18% or less, even more preferably 16% or less, particularly preferably 14% or less at a thickness of 1.0 mm.

When the linear light transmittance of the alumina sintered body is higher than 20% at a thickness of 1.0 mm, it may lead to excessively high translucency (transmissivity for linear light) in the incisal region of dental prostheses, failing to provide aesthetics suited for the tip (incisal edge) of teeth.

The content of alumina, bluish colorants, and sintering aids in an alumina sintered body of the present invention is the same as that in the composition and/or pre-sintered body to be fabricated into a sintered body.

An alumina sintered body of the present invention has a translucency (ΔL) of preferably 5 or more, more preferably 10 or more, even more preferably 15 or more, particularly preferably 20 or more. The translucency (ΔL) takes a lightness value L* (color space) of L*a*b* color system (JIS Z 8781-4:2013), and it is a value after subtracting a second L* value from a first L* value, where the first L* value is an L* value measured for a 1.2 mm-thick sample against a white background, and the second L* value is an L* value measured for the same sample against a black background.

Samples for the measurement of translucency (ΔL) can be prepared as follows, for example. First, granules (composition) are press molded to provide a thickness of 1.2 mm for the sintered body to be produced, and subsequent CIP molding forms, for example, a disc-shaped molded body measuring 19 mm in diameter. The molded body can then be fired under predetermined firing conditions, and a sintered body, or a sample, having a thickness of 1.2 mm can be prepared by polishing the surface at #2000. For the measurement of L* values, a colorimeter (for example, CE100, analysis software Crystaleye manufactured by Olympus Corporation) can be used to measure L* values against black and white backgrounds after applying a contact liquid to a sample surface. The contact liquid may be, for example, one having a measured refractive index nD of 1.60 at 589 nm wavelength (sodium D-line).

The b* value is preferably -8.0 to 14.2, more preferably -7.0 to 14.0, even more preferably -6.0 to 13.8 in a sintered body of 1.2 mm thickness.

In certain preferred embodiments, the b* value is preferably 0 to 14.0, more preferably 0.1 to 13.9, even more preferably 0.2 to 13.8.

The method of measurement of b* value is as described in the EXAMPLES section below.

An alumina sintered body of the present invention may be a molded body of a predetermined shape. For example, the sintered body may have a disc (discotic) shape, a cuboidal shape, or a shape of a dental product (for example, a crown shape).

The methods of production of the alumina compositions, granules, powders, molded bodies, pre-sintered bodies, cut or ground workpieces, and sintered bodies described in the present invention are not limited those described above, and a variety of known methods are applicable, except where specified otherwise.

The present invention encompasses embodiments combining the foregoing features in various ways within the technical idea of the present invention, provided that the present invention can exhibit its effects.

In the present invention, the upper limits and lower limits of numeric ranges (for example, ranges of contents of components, ranges of various elements (such as the average primary particle diameter), and ranges of physical properties) can be appropriately combined.

An alumina pre-sintered body of the present invention can be suitably used for alumina processed products requiring high transparency after firing. Examples include dental materials, optical fiber cable connectors, smartphone casings, semiconductors, liquid crystal manufacturing fixtures, separation membranes, translucent tubes for high-pressure sodium lamps, watch windows, polishing materials for magnetic tapes, display materials, and battery electrode materials.

Specifically, applications requiring precise machining at the pre-sintered body stage are well-suited, with dental applications particularly suited due to the required processability, translucency, and strength.

### EXAMPLES

The present invention is described below in greater detail. It should be noted, however, that the present invention is in no way limited by the following Examples, and various changes may be made by a person with ordinary skill in the art within the technical idea of the present invention.

### [Measurement of Average Primary Particle Diameter]

The pre-sintered bodies obtained in the Examples and Comparative Examples below were used to prepare surface images, using a scanning electron microscope (VE-9800 manufactured by Keyence under this trade name). For the measurement of particle diameter, the SEM image with indicated primary particles was binarized with image analysis software (Image-Pro Plus manufactured by Hakuto Co., Ltd. under this trade name), and particles were recognized from the field (region) after indicating grain boundaries on individual crystal grains in the image. In areas with unclear grain boundaries, a reduce filter was applied to the region until each region reduced to a single or multiple points, and Voronoi polygons were created by using these points as generators of Voronoi polygons. Lines were drawn that connect the midpoints between two adjacent generators, and these lines were superimposed on the original particle image to separate adjacent particles. For example, in the case where image processing produced a particle that looks like a gourd, the particle was separated into two by assuming that two adjoining circular particles are seen as a single particle. In a processing file recognizing primary particle diameter, "Diameter" was selected from "Count/size dialog" to find the distribution (n = 4). Specifically, a mean value of particle diameters (primary particle diameters) measured in each field with the image analysis software (Image-Pro Plus) was determined for four fields from one sample. FIG. 1 shows an electron micrograph of the pre-sintered body according to Example 1.

### [Measurement of Sintering Aid Content]

The pre-sintered bodies obtained in the Examples and Comparative Examples below were used as samples.

For the measurement, a field-emission scanning electron microscope (FE-SEM Reglus 8220, manufactured by Hitachi High-Tech Corporation) and an energy dispersive X-ray analyzer (Aztec Energy X-Max 50, manufactured by Oxford Instruments) were used under the following conditions.
Measurement magnification: 5,000×
Analysis mode: surface analysis
Acceleration voltage: 5 kV
Working distance: 15 mm ± 1 mm
X-ray extraction angle: 30 degrees
Dead time: 7%
Measurement time: 100 seconds

### [Measurement of Pigment Dispersibility in Pre-Sintered Body]

The pre-sintered bodies obtained in the Examples and Comparative Examples below were used as samples.

For the measurement, a field-emission scanning electron microscope (FE-SEM Reglus 8220, manufactured by Hitachi High-Tech Corporation) and an energy dispersive X-ray analyzer (Aztec Energy X-Max 50, manufactured by Oxford Instruments) were used under the following conditions.
Measurement magnification: 20,000×
Analysis mode: line analysis
Acceleration voltage: 5 kV
Working distance: 15 mm ± 1 mm
X-ray extraction angle: 30 degrees
Dead time: 7%
Measurement time: 100 seconds

In the region where alumina was detected by line analysis, the physical distance representing the concentration difference of the elements constituting the pigment was determined by measuring the physical distance between a single maximum value and the surrounding minimum values, and averaging the physical distances between adjacent maximum and minimum values in the waveform representing the detected amounts obtained through line analysis for the constituent elements of the pigment.

The field was adjusted in different positions so that there were a total of 20 connected fields (10 fields × 2 fields), with a distance of 50 µm within the connected field in line analysis. Analysis was conducted by measuring twenty locations, and the average value from the resulting waveforms was determined as the physical distance.

The dispersibility was determined as "Good" when the physical distance was within 50 µm, and "Poor" when it exceeded 50 µm.

### [Measurement Method of Average Crystal Grain Size of Sintered Body]

The sintered bodies obtained in the Examples and Comparative Examples were photographed to capture surface images, using a scanning electron microscope (VE-9800 manufactured by Keyence under this trade name). The average crystal grain size was calculated through image analysis after indicating grain boundaries on individual crystal grains in the image.

For the measurement of average crystal grain size, the captured SEM image was binarized with image analysis software (Image-Pro Plus manufactured by Hakuto Co., Ltd. under this trade name), and particles were recognized from the field (region) by adjusting the brightness range to provide clear grain boundaries.

The crystal grain size from Image-Pro Plus is the average of the measurements of the length of a line segment connecting the contour line and passing through the center of gravity determined from the contour line of the crystal grain, conducted at 2-degree intervals with the center of gravity as the central point.

The measurement of crystal grain size was conducted for all particles not extending beyond the edges of the SEM photographic image (3 fields) of the alumina sintered body.

The average crystal grain diameter was calculated from the crystal grain size of each particle and the number of crystal grains. The resulting arithmetic average diameter was then determined as the average crystal grain size of the sintered body.

Note that particles not extending beyond the edges of the image are particles excluding those with contour lines extending beyond the screen of the SEM photographic image (particles with their contour lines interrupted by the boundary lines at the top, bottom, left, and right). To select the crystal grain size of all particles not extending beyond the edges of the image, the option in Image-Pro Plus was used that excludes all particles lying on the boundary lines.

### [Measurement Method of Translucency of Sintered Body]

The sintered body obtained in each Example and Comparative Example was ground into a plate sample with a thickness of 1.2 mm. The sample was then measured for first lightness (Lw*) and second lightness (L_{B}*) by using a spectrophotometer (manufactured by Olympus Corporation under the trade name Crystaleye) in 7-band measurement mode with an LED light source. The first lightness (Lw*) is the lightness resulting from the measurement of chromaticity against a white background, and the second lightness (L_{B}*) is the lightness resulting from the chromaticity measurement of the same sample conducted against a black background using the same measurement device in the same measurement mode with the same light source. The difference between these values (ΔL = (Lw*) - (L_{B}*)) was determined as the translucency (ΔL) (a mean value for n = 3).

The white background means the white part of the hiding-power test paper described in JIS K 5600-4-1:1999, Part 4, Section 1, and the black background means the black part of the hiding-power test paper.

The preferred translucency (ΔL) of the alumina sintered body is 5 or more, more preferably 10 or more, even more preferably 15 or more, particularly preferably 20 or more.

### [Measurement Method of Total Transmittance and Linear Light Transmittance of Sintered Body]

The sintered bodies of Examples and Comparative Examples were measured for total transmittance and linear light transmittance in each layer. The measurement was individually conducted for each layer of the alumina sintered bodies and zirconia sintered bodies fabricated using the following method.

First, a molded body was fabricated from the raw material powder of each layer in Examples and Comparative Examples by pressing the powder in a die having a diameter of 30 mm. Here, the raw material powder was introduced into the die in adjusted amounts to ensure the production of alumina sintered bodies and zirconia sintered bodies with a thickness of 1.0 mm.

The molded body was heated from room temperature at 10°C/min. Following a 2-hour retention at 500°C for the purpose of debinding organic components, the molded body was heated at 10°C/min, and maintained for 6 hours at a highest pre-sintering temperature of 700°C (for the production of alumina pre-sintered bodies) or 1,000°C (for the production of zirconia pre-sintered bodies). The molded body was then gradually cooled at -0.4°C/min to yield alumina pre-sintered bodies and zirconia pre-sintered bodies.

Subsequently, the alumina pre-sintered bodies and zirconia pre-sintered bodies were fired for 2 hours at the highest sintering temperatures presented in Table 2, fabricating alumina sintered bodies and zirconia sintered bodies. The alumina sintered bodies and zirconia sintered bodies were polished to a mirror finish on both sides to prepare alumina sintered bodies and zirconia sintered bodies with a thickness of 1.0 mm. These alumina sintered bodies and zirconia sintered bodies were then measured with a turbidimeter (Haze Meter NDH 4000 manufactured by Nippon Denshoku Industries Co., Ltd.). The measurement was conducted according to ISO 13468-1 and JIS K 7361-1:1997, and the measured values were averaged for n = 3. The results are presented in Table 2.

### [Chromaticity Measurement (b* value)]

The b* value was determined by measuring the chromaticity (color space) in the L*a*b* color system (JIS Z 8781-4:2013, Color Measurements - Part 4: CIE 1976 L*a*b* color space) based on measurements with a dental colorimeter (Crystaleye CE100-DC/JP, 7-band LED light source, manufactured by Olympus Corporation) and the analysis software Crystaleye (manufactured by Olympus Corporation). The measured b* values were used to calculate the average value (a mean value for n = 3).

The measurement was conducted for alumina sintered bodies and zirconia sintered bodies with a diameter of 14 mm and a thickness of 1.2 mm.

The preferred b* value is -8.0 to 14.2, more preferably -7.0 to 14.0, even more preferably -6.0 to 13.8.

### [Aesthetics at Incisal Edge]

The alumina pre-sintered bodies and zirconia pre-sintered bodies fabricated for Examples and Comparative Examples using the method described above were fired for 2 hours at the sintering temperatures presented in Table 2. This resulted in the fabrication of alumina sintered bodies and zirconia sintered bodies with a diameter of 14 mm and a thickness of 1.2 mm. The disc-shaped alumina sintered bodies and zirconia sintered bodies were visually evaluated using the criteria below.

The alumina sintered bodies and zirconia sintered bodies were determined as fulfilling the criteria when at least three out of four observers agreed that the following criteria were met. The results are presented in Table 2.

Note that the observers observed the alumina sintered bodies and zirconia sintered bodies from a distance of 30 cm from the eyes.

### <Evaluation Criteria>

Good: Demonstrates the same level or better translucency than that of natural teeth, with surface reflection comparable to natural teeth. With addition of appropriate coloring components or similar additives, the sintered bodies can faithfully reproduce the appearance of natural teeth.

Moderate: Demonstrates the same level or better translucency than that of natural teeth, but with excessive surface reflection, failing to faithfully reproduce the appearance of natural teeth.

Poor: Low translucency, unable to reproduce the appearance of natural teeth even with the addition of coloring components or similar additives.

### <Example 1>

After weighing 100 g of α-alumina raw material NXA-100 (manufactured by Sumitomo Chemical Co., Ltd.), 0.83 g of magnesium chloride hexahydrate (an equivalent to 1,000 ppm by mass in terms of a Mg element), and 0.4 mg of cobalt chloride hexahydrate (an equivalent to 1 ppm by mass in terms of a Co element), these were introduced into 0.7 L of ethanol and ultrasonically dispersed.

These were introduced into a rotary container along with alumina beads, and the alumina raw material, including aggregated particles, was pulverized with a ball mill until the desired average primary particle diameter was achieved through mixing and crushing. The average primary particle diameter was measured by volume with ultrasonic waves being applied after a slurry diluted with ethanol was subjected to 30 minutes of ultrasonication, using a laser diffraction/scattering particle size distribution analyzer (manufactured by Horiba Ltd. under the trade name Partica LA-950). The desired slurry with hardly any secondary aggregation was obtained after about 1 hour of ball milling.

Subsequently, an organic binder was added to the slurry. An aqueous acrylic binder, serving as the organic binder, was added in an amount of 2 mass% with respect to the α-alumina raw material. The mixture underwent stirring with rotary vanes for 24 hours. After stirring, the slurry underwent drying and granulation with a spray dryer, yielding granules. The granules had an average particle diameter of 40 µm. The powder formed by these granules was introduced into a cuboidal die of a predetermined size, and uniaxially pressed at 150 MPa to yield a molded body. The molded body was placed in an electric furnace, and heated from room temperature at 10°C/min. Following a 2-hour retention at 500°C for the purpose of debinding organic components, the molded body was heated, maintained for 6 hours at a highest pre-sintering temperature of 700°C under atmospheric pressure, and gradually cooled at -0.4°C/min to yield a pre-sintered body.

Subsequently, the pre-sintered body was cut by machining to prepare a workpiece of 1.5 mm thickness. The workpiece was heated from room temperature at 10°C/min, retained at a highest sintering temperature of 1,400°C for 2 hours under atmospheric pressure, and gradually cooled at -0.4°C/min to yield a sintered body.

### <Examples 2 to 5>

The same procedure employed in Example 1 was used to obtain pre-sintered bodies and sintered bodies, with the exception that NXA-100, NXA-150, or AKP-53 (manufactured by Sumitomo Chemical Co., Ltd.) was used as α-alumina raw material, and that cobalt chloride hexahydrate was added in an amount equivalent to 10, 20, or 50 ppm by mass in terms of a Co element.

### <Example 6>

After weighing 100 g of α-alumina raw material NXA-100 (manufactured by Sumitomo Chemical Co., Ltd.), 0.83 g of magnesium chloride hexahydrate (an equivalent to 1,000 ppm by mass in terms of a Mg element), and 8 mg of cobalt chloride hexahydrate (an equivalent to 20 ppm by mass in terms of a Co element), these were introduced into 0.7 L of ethanol and ultrasonically dispersed.

These were introduced into a rotary container along with alumina beads, and the alumina raw material, including aggregated particles, was pulverized with a ball mill until the desired average primary particle diameter was achieved through mixing and crushing. The average primary particle diameter was measured by volume with ultrasonic waves being applied after a slurry diluted with ethanol was subjected to 30 minutes of ultrasonication, using a laser diffraction/scattering particle size distribution analyzer (manufactured by Horiba Ltd. under the trade name Partica LA-950). The desired slurry with hardly any secondary aggregation was obtained after about 1 hour of ball milling.

The slurry underwent stirring in a 2 L beaker using rotary vanes at 200 rpm for 1 hour. Following an abrupt cessation of rotary vanes movement, the slurry was left to stand for 15 minutes. Settling of white particles was visible at the bottom of the beaker, and the supernatant appeared cloudy. The upper third of the slurry within the beaker was extracted as the slurry for Example 6. In Table 1, the distinction between slurries is based on elutriation, with the raw material of Example 6 labeled as "Above NXA-100 elutriation".

The same technique and procedure employed in Example 1 were used to prepare pre-sintered bodies and sintered bodies from the slurry.

### <Comparative Example 1>

The pre-sintered body and sintered body were obtained using yttria-stabilized zirconia, instead of NXA-100 used as α-alumina raw material. The yttria-stabilized zirconia was prepared using the following method.

A zirconia (zirconium oxide, ZrO₂) powder and a yttria (yttrium oxide, Y₂O₃) powder were used to prepare a mixture with 5.5 mol% of yttria relative to the total mole of zirconia and yttria. The mixture was added into water to prepare a slurry. The slurry underwent wet pulverization with a ball mill through mixing until it had an average particle diameter of 0.13 µm or less. After pulverization, the slurry was dried with a spray dryer, and the resulting powder was fired at 950°C for 2 hours to prepare a powder (primary powder).

The primary powder was added into water to prepare a slurry. The slurry underwent wet pulverization with a ball mill through mixing until it had an average particle diameter of 0.13 µm or less. After adding a binder to the pulverized slurry, the slurry was dried with a spray dryer to prepare a powder (secondary powder). The secondary powder prepared was used as the raw material powder to produce the zirconia pre-sintered body of Comparative Example 1.

The zirconia pre-sintered body was produced as follows. First, the raw material powder was filled into a die having inside dimensions of 20 mm × 25 mm, and uniaxially pressed at a pressure of 150 MPa to prepare a molded body. The molded body was placed in an electric furnace, and heated from room temperature at 10°C/min. Following a 2-hour retention at 500°C for the purpose of debinding organic components, the molded body was heated, maintained for 6 hours at a highest pre-sintering temperature of 1,000°C, and gradually cooled at -0.4°C/min to yield a zirconia pre-sintered body.

Subsequently, the pre-sintered body was cut by machining to prepare a workpiece of 1.5 mm thickness. The workpiece was heated from room temperature at 10°C/min, retained at a highest sintering temperature of 1,550°C for 2 hours, and gradually cooled at -0.4°C/min to yield a sintered body.

### <Comparative Example 2>

The pre-sintered body and sintered body were obtained using the same procedure employed in Comparative Example 1, except for the following changes. The yttrium oxide used in Comparative Example 1 was replaced with magnesium chloride. Magnesium chloride hexahydrate was added in an amount equivalent to 1,000 ppm by mass in terms of a Mg element. Cobalt chloride hexahydrate was added in an amount equivalent to 50 ppm by mass in terms of a Co element. The highest sintering temperature was varied as shown in Table 2.

### <Comparative Example 3>

The pre-sintered body and sintered body were obtained using the same procedure employed in Example 2, except that AA-03 (manufactured by Sumitomo Chemical Co., Ltd.) was used as α-alumina raw material, instead of NXA-100.

### <Comparative Example 4>

The pre-sintered body and sintered body were obtained using the same procedure employed in Example 2, except that magnesium chloride hexahydrate was not added as a sintering aid.

### <Comparative Example 5>

The pre-sintered body and sintered body were obtained using the same procedure employed in Example 2, except that magnesium chloride hexahydrate, serving as a sintering aid, was added in an amount equivalent to 10,000 ppm by mass in terms of a Mg element.

The results for Examples and Comparative Examples are presented in Tables 1 and 2.

**[Table 1]**

| | Pre-sintered body | | | | | | |
|---|---|---|---|---|---|---|---|
| | Main ingredient | Average primary particle diameter [n m] | Sintering aid | | Bluish colorant | | |
| | | | Type | Content¹) [ppm] | Type | Content²) [ppm] | Uniform dispersibility |
| Ex. 1 | NXA-100 | 100 | MgCl₂ | 1000 | CoCl | 1 | Good |
| Ex. 2 | NXA-100 | 100 | MgCl₂ | 1000 | CoCl | 10 | Good |
| Ex. 3 | NXA-150 | 150 | MgCl₂ | 1000 | CoCl | 20 | Good |
| Ex. 4 | NXA-150 | 150 | MgCl₂ | 1000 | CoCl | 50 | Good |
| Ex. 5 | AKP-53 | 170 | MgCl₂ | 1000 | CoCl | 50 | Good |
| Ex. 6 | Above NXA-100 elutriation | 40 | MgCl₂ | 1000 | CoCl | 20 | Good |
| Com. Ex. 1 | Zirconia | 130 | Y₂O₃ | 100000 | CoCl | 50 | Good |
| Com. Ex. 2 | Zirconia | 130 | MgCl₂ | 1000 | CoCl | 50 | Good |
| Com. Ex. 3 | AA-03 | 400 | MgCl₂ | 1000 | CoCl | 10 | Good |
| Com. Ex. 4 | NXA-100 | 100 | - | - | CoCl | 10 | Good |
| Com. Ex. 5 | NXA-100 | 100 | MgCl₂ | 10000 | CoCl | 10 | Good |

| | | | | | | | |
|---|---|---|---|---|---|---|---|
| 1) An amount in terms of a Mg element or Y element. 2) An amount in terms of a Co element. | | | | | | | |

**[Table 2]**

| | Sintered body | | | | | | |
|---|---|---|---|---|---|---|---|
| | Highest sintering temperature [°C] | Average crystal grain size [µm] | Linear light transmittance [%] | Translucency (ΔL) [-] | Total transmittance [%] | b* value [-] | Evaluation result for aesthetics at incisal edge |
| Ex. 1 | 1400 | 0.9 | 9.5 | 28.3 | 61.2 | 13.8 | Good |
| Ex. 2 | 1400 | 1.2 | 6.2 | 22.1 | 52.2 | 11.6 | Good |
| Ex. 3 | 1400 | 1.5 | 4.1 | 19.7 | 45.5 | 9.5 | Good |
| Ex. 4 | 1400 | 1.8 | 2.1 | 15.5 | 43.6 | 0.8 | Good |
| Ex. 5 | 1400 | 2.2 | 1.2 | 14.3 | 40.5 | 0.6 | Good |
| Ex. 6 | 1400 | 1.1 | 6.4 | 22.8 | 53.1 | 10.2 | Good |
| Com. Ex. 1 | 1550 | - | 0.5 | 15.5 | 48.0 | 0.8 | Moderate |
| Com. Ex. 2 | 1400 | - | 0.1 | 2.3 | 29.1 | 1.5 | Poor |
| Com. Ex. 3 | 1400 | 8.1 | 0.0 | 3.5 | 26.5 | 8.2 | Poor |
| Com. Ex. 4 | 1400 | 5.3 | 0.1 | 3.2 | 12.3 | 9.9 | Poor |
| Com. Ex. 5 | 1400 | 1.1 | 0.5 | 9.8 | 31.4 | 14.8 | Poor |

As demonstrated above, it was possible in Examples 1 to 6 to reduce yellowing in the sintered bodies after sintering, and achieve high translucency and high linear light transmittance in the sintered bodies even after sintering under atmospheric pressure, providing highly aesthetic alumina sintered bodies suited for dental applications.

### INDUSTRIAL APPLICABILITY

An alumina pre-sintered body for dental use of the present invention can be used to provide alumina sintered bodies for dental use having high linear light transmittance and reduced yellow tint. An alumina pre-sintered body for dental use of the present invention transforms into a highly aesthetic alumina sintered body suited for the appearance of the incisal edge, and is particularly suitable as dental prostheses for incisors or canines.

## Claims

1. An alumina pre-sintered body for dental use, comprising alumina particles with an average primary particle diameter of 30 to 300 nm, a sintering aid, and a bluish colorant,
wherein the content of the sintering aid is 10 to 5,000 ppm.

2. The alumina pre-sintered body for dental use according to claim 1, wherein the bluish colorant comprises a cobalt component.

3. The alumina pre-sintered body for dental use according to claim 2, wherein the content of the cobalt component is 60 ppm or less.

4. The alumina pre-sintered body for dental use according to claim 2 or 3, wherein the cobalt component is a component derived from a salt and/or a complex.

5. The alumina pre-sintered body for dental use according to any one of claims 1 to 4, which shows a linear light transmittance of 0.8% or more as a sintered body of 1.0 mm thickness fired under atmospheric pressure without a hot isostatic pressing process.

6. The alumina pre-sintered body for dental use according to any one of claims 1 to 5, which shows a b* value of -8.0 to 14.2 as a sintered body of 1.2 mm thickness fired under atmospheric pressure without a hot isostatic pressing process.

7. The alumina pre-sintered body for dental use according to any one of claims 1 to 6, wherein the sintering aid comprises at least one element selected from the group consisting of a Group 2 element, Ce, Zr, and Y

8. The alumina pre-sintered body for dental use according to any one of claims 1 to 7, wherein the alumina particles comprise α-alumina with a purity of 99.5% or more.

9. A dental mill blank comprising a portion with the alumina pre-sintered body of any one of claims 1 to 8.

10. The dental mill blank according to claim 9, wherein said portion is a portion with the highest translucency.

11. A method for producing an alumina pre-sintered body for dental use, comprising the step of allowing a bluish colorant to uniformly adhere to alumina particles,
wherein the alumina pre-sintered body for dental use comprises alumina particles with an average primary particle diameter of 30 to 300 nm, a sintering aid, and a bluish colorant, and
the content of the sintering aid is 10 to 5,000 ppm.

12. The method for producing an alumina pre-sintered body for dental use according to claim 11, wherein the step of allowing a bluish colorant to uniformly adhere to alumina particles comprises the step of dispersing α-alumina particles in a dispersion medium with the bluish colorant dissolved in the dispersion medium.

13. The method for producing an alumina pre-sintered body for dental use according to claim 11 or 12, which comprises firing at 600°C or more and 1,200°C or less under atmospheric pressure.

14. An alumina sintered body for dental use having an average crystal grain size of 0.3 to 8.0 µm and comprising a sintering aid and a bluish colorant,
wherein the content of the sintering aid is 10 to 5,000 ppm.

15. The alumina sintered body for dental use according to claim 14, wherein the bluish colorant comprises a cobalt component.

16. The alumina sintered body for dental use according to claim 15, wherein the content of the cobalt component is 60 ppm or less.

17. The alumina sintered body for dental use according to claim 15 or 16, wherein the cobalt component is a component derived from a salt and/or a complex.

18. The alumina sintered body for dental use according to any one of claims 14 to 17, which has a linear light transmittance of 0.8% or more at a thickness of 1.0 mm.

19. The alumina sintered body for dental use according to any one of claims 14 to 18, which has a b* value of -8.0 to 14.2 as a sintered body of 1.2 mm thickness.

20. The alumina sintered body for dental use according to any one of claims 14 to 19, wherein the sintering aid comprises at least one element selected from the group consisting of a Group 2 element, Ce, Zr, and Y
